# EUROPEAN PATENT SPECIFICATION

(11) **EP 0 778 826 B1**
(45) Date of publication and mention of the grant of the patent: **03.11.1999**
(21) Application number: 95928583.4
(22) Date of filing: 17.08.1995
(51) Int. Cl.: C07C 409/24, C07C 409/40

(54) **PROCESS FOR SOLID PERACID MANUFACTURE**
VERFAHREN ZUR HERSTELLUNG VON FESTEN PERSÄUREN
PROCEDE DE PRODUCTION DE PERACIDES SOLIDES

(30) Priority: 03.09.1994 GB 9417723
(43) Date of publication of application: 18.06.1997
(73) Proprietor: SOLVAY INTEROX LIMITED, Warrington, Cheshire WA4 6HB (GB)
(72) Inventor: CARR, Graham, Woolton Liverpool L25 5HR (GB); JAMES, Alun, Pryce, Blundellsands Liverpool L23 8SW (GB); McADAM, Johnathan, St. Helens Merseyside WA9 4NN (GB); SANDERSON, William, Ronald, Warrington Cheshire WA5 2PE (GB)
(74) Representative: Vande Gucht, Anne
(86) International application number: GB9501956
(87) International publication number: WO9607639

(56) References cited:
- EP-A- 0 612 726
- WO-A-90/14336
- WO-A-92/11238

## Description

The present invention relates to a process for the manufacture of solid peracids and more particularly to the manufacture of particulate peracids to permit their isolation and recovery from an acidic reaction medium. The present invention also relates to peracid products having modified and/or improved properties and/or morphology.

Peracids, sometimes alternatively referred to as peroxyacids, as a class represent a range of potentially useful materials, since they are powerful oxidants, are effective wide spectrum biocides and are effective bleaches of stains, both under industrial conditions and in the domestic environment. In 3a number of formulations, the peroxyacids are employed as described for employment in solid, particulate form. In consequence, it is desirable to devise processes for the manufacture of peracids that result in an isolatable particulate solid. It is inherently advantageous to devise processes which can render easier or quicker recovery of solid peracid from a reaction medium, and/or control the particle size and distribution of the peracid solids. Furthermore, it is desirable to obtain peracids which enjoy in the solid state modified and/or improved physical properties and/or a new morphology.

In one process for making peracids, an organic carboxylic acid substrate is brought into reactive contact with hydrogen peroxide in the presence of a strong acid such as a mineral acid like sulphuric acid or a corresponding organic acid such as methanesulphonic acid. Many of the carboxylic acid substrates are solid at convenient operating temperatures, that are often selected and controlled in the range of from 5 to 45°C and are poorly soluble in aqueous media. Likewise, many of the resultant peracids are also poorly soluble in aqueous media. Several variations have been described in current literature in the manner in which the carboxylic acid, the hydrogen peroxide and the strong acid are brought into contact with each other. In many of the variations, steps are taken to promote the reaction in the liquid phase, for example by dissolving the substrate in at least a fraction of the strong acid before it is brought into contact with the hydrogen peroxide. In other variations, the hydrogen peroxide is premixed with at least a fraction of the strong acid. Whatever the technique for mixing the reagents, it has been recognised since at least USP 2813896 (Krimm) that one important parameter is the ratio of strong acid to carboxylic acid, and that a high ratio of strong acid to carboxylic acid favours high conversion to the percarboxylic acid. This can be expressed as a high molar or weight ratio as in an article by Y L Zherebin et al in Zorka, vol. 8, p41-44 (1972) that prefers a 20 fold molar excess of sulphuric acid over dicarboxylic acid. Alternatively, it can be expressed as the reaction mixture containing a high proportion of strong, e.g. sulphuric acid, often of over 60% w/w and in many instances up to about 80% w/w. The proportion of sulphuric acid compared with the total weight of sulphuric acid and water is sometimes alternatively called the A value.

Reaction mixtures having high A values tend to be able to dissolve both the carboxylic acid substrate faster and/or the resultant peracid product to a greater extent than can low A value reaction mixtures. As a consequence, a fraction of the peracid product, and in many instances a considerable fraction of the product remains in solution when the reaction is finished.

One method of improving the extent of recovery of solid peracid from a concentrated acidic solution is to lower the A value of the reaction mixture by introduction of water or ice, an operation that is often called quenching. In the course of conducting experimentation into the manufacture of peracids and especially diperacids that are only poorly soluble in aqueous media in the region of neutral pH, it has been found that the method of quenching can affect to a significant extent the properties of the resultant solid product. These properties can include the physical properties of the solid that is produced, which in turn affect the rate at which the solids can be separated from the supernatant liquor in conventional separation methods, such as filtration and the proportion of liquor retained by the solids. It is inherently desirable to devise precipitation processes that produce a readily filterable product, because products that are poorly filterable can markedly increase product recovery times and/or require excessively large filtration equipment, both of which can increase significantly manufacture costs. Excessive liquor retention in the solids not only leads to potentially increased drying costs but also increases either the time taken for washing/purification and hence its costs or the level of residual and destabilising acidic impurity in the product. On the other hand, the chemical properties of the resultant peracid are also of considerable practical importance, such as the extent of reversion or partial reversion to either the carboxylic acid substrate or the half carboxylic acid half peracid in the case of a diperacid, since they determine the extent of net conversion of the precursor to the peroxyacid and the effective base cost of the product per unit amount of peroxyacid available oxygen (avox).

As a general tendency, in the course of observing the dilution/recovery of the same peroxyacid under a range of conditions, it has been found that the commonly disclosed technique of dropping the reaction mixture containing sulphuric acid into a substantial volume of ice/water diluent such as double the reaction mixture volume dilutes to a desirable extent, but tends to produce an extremely fine particulate product that can blind filters and is slower to dewater. The effect is observable not only on the initial filtration but especially during subsequent washing to remove undesired acid impurities. On the other hand, it has been observed that a progressive slow dilution of the mixture with the same volume of diluent tends to alter the morphology of the product and to impair the chemical purity of the product, the latter effect possibly as a result of acid catalysed hydrolysis (reequilibration) of the product.

It is an object of the present invention to devise a process for the preparation of a peroxyacid in solid form from a solution thereof in a reaction mixture of high A value which avoids and/or ameliorates the twin problems of excessively slow rate of filtration and excessively impaired peracid purity.

In accordance with a first aspect of the present invention there is provided a process for the manufacture of particulate poorly water-soluble peroxyacid in which the peroxyacid is present in an aqueous solution of a strong acid having an A value of at least 0.6 which is brought into mixture with diluent aqueous material in an amount sufficient that the peroxyacid precipitates out of solution characterised in that contact with diluent aqueous material selected from water or dilute aqueous solution of strong acid and/or hydrogen peroxide is conducted in the presence of an effective amount of at least one surfactant.

By incorporating an effective amount of a surfactant during the process of diluting the solution of peroxyacid in the reaction mixture, it is possible to improve the balance of properties of the resultant particulate peroxyacid solid, including its particle size, shape and size distribution and its residual peracid avox (available oxygen) content. Where the peroxyacid is a diperacid, the balance includes the distribution between diperacid, monoperacid and dicarboxylic acid species in the separable product.

The solution of peroxyacid before dilution will normally comprise a reaction mixture obtained by reacting a peroxyacid precursor, which usually would comprise the corresponding carboxylic acid, but which could alternatively comprise the corresponding anhydride or less commonly an ester, with concentrated aqueous hydrogen peroxide in a strong acid reaction medium. The reaction medium is advantageously a mineral acid such as especially sulphuric acid, by virtue of its ready availability and high acid strength or can if desired comprise an organic derivative such as methanesulphonic acid. The selection of the reaction conditions is at the discretion of the peroxyacid manufacturer, taking into account which peroxyacid is being made. Such conditions include specifically the acid strength of the reaction mixture, often referred to as the A value, which the weight proportion represented by the strong, e.g. sulphuric acid, of the total weight of sulphuric acid, plus water in the reaction mixture. The A value in the reaction mixture is normally at least 0.6 and is often not higher than 0.9. The inverse relationship of a preferred A value to the inherent solubility of the precursor carboxylic acid is disclosed for example in International Publication No WO 90/14336. In general, it is preferred to employ an A value of between 0.7 and 0.8 to balance the twin constraints of operational safety and reactivity.

There are several variations in the methods of bringing together peroxyacid precursor and the hydrogen peroxide reactants in order to produce a reaction mixture containing eventually peroxyacid, all of which can be employed at the discretion of the manufacturer for the formation of a peroxyacid solution from which particulate peroxyacid product can be recovered by a process according to the present invention, and many of which variations have been described in published literature.

Such variations include the dissolution of the peroxyacid precursor in all or a fraction of the strong acid (e.g. sulphuric acid) and the slow addition thereto of concentrated hydrogen peroxide. In other variations, a fraction of the sulphuric acid is premixed with the aqueous hydrogen peroxide solution which is then mixed with the solution of precursor in sulphuric acid, for example in a controlled manner such as described in aforesaid WO 90/14336 (e.g. Example 8) or possibly in a continuous process. In other variations, the peroxyacid precursor is employed in the form of a particulate solid which is progressively introduced into the premixture of sulphuric acid and hydrogen peroxide.

In a further variation employing a particulate carboxylic acid precursor, which variation is the subject of a copending PCT application, the reaction medium is produced in two stages, in the first of which a reaction mixture of lower A value is produced which is increased to a higher A value in the presence of or immediately prior to contact with the particulate carboxylic acid.

In the foregoing reactions, the reaction temperature is usually controlled to within the range of from 5°C to 50°C, and in many instances from 15 to 35°C. The hydrogen peroxide is usually employed in excess over the stoichiometric amount, ie at greater than 1 mole per equivalent mole of carboxylic acid (or corresponding anhydride or ester) precursor and often from 2 to 5 moles per equivalent mole. Its concentration in the liquid phase of the reaction medium is often initially from 3 to 20% by weight, depending in part on the ratio of the total weight of hydrogen peroxide/sulphuric acid/water to the peroxyacid precursor. Such ratio is normally at least 4:1 and usually not higher than 30:1. Although the selection of that ratio is at the discretion of the peroxyacid manufacturer, for the manufacture of the particularly preferred aliphatic diperoxyacids containing an embedded phenylene group described herein, a preferred range is from 5:1 to 9:1 and especially when employing carboxylic acid precursor in particulate form.

In accordance with recognised procedures, the reaction mixture can contain, if desired, a small fraction such as 0.1 to 5 gpl of known stabiliser(s) for hydrogen peroxide and/or peroxyacids such as picolinic acid, dipicolinic acid, and/or organopolyphosphonates.

As a result of the peroxidation process, there is produced a reaction mixture containing peroxyacid in solution. The invention process is applicable particularly to reaction mixtures which are free from solids before quenching, be they peroxyacid or peroxyacid precursor.

Depending on the mole ratio of the reactants employed, the product obtained from the reaction between hydrogen peroxide and dicarboxylic acid can comprise a proportion of monoperacid as well as diperacid, the proportion of monoperacid varying inversely to variation in the mole ratio of hydrogen peroxide to dicarboxylic acid. The present invention is also applicable to the recovery of product containing a substantial proportion of monoperacid.

Preferably, the peroxyacid that is produced in particulate form by a process according to the present invention is a diperoxyacid of formula 1 HO₃C-R'-(NR)ₐ-CO-(NR)_{b}-R"-(NR)_{c}-CO-(NR)_{d}-R'"-CO₃H in which R represents hydrogen, alkyl, aryl, or alkaryl or aralkyl to 12 carbon atoms, a + b = 1, c + d = 1, R' and R"' each represents a methylene or polymethylene group of from 1 to 10 carbons that is optionally substituted by an aryl group and R" represents an arylene or a polymethylene group containing up to 10 carbons, or together with an adjacent nitrogen an aromatic heterocyclic group. The present invention also applies to monoperoxyacids corresponding to the diperacids of formula 1 above, in which one of the peroxyacid groups is a carboxylic acid.

The number of carbons in the compound of formula 1 is often selected in the range of from 12 to 30 and in some especially preferred compounds is from 18 to 22, and particularly 20. It is especially preferred for R to represent hydrogen and particularly preferable for a and d to each equal 1, ie b and c to equal 0 and especially if R" is arylene. In compounds according to formula 1, the aryl group R, R' or R"' or arylene group R" can, if desired, be substituted by a halo, alkyl group or further aryl group, ie examples of groups which are not themselves oxidised or removed during the reaction process in which the peroxyacid is produced and in a number of compounds are preferably respectively phenyl and phenylene. R' and R"' are each preferably selected from the group of from trimethylene up to hexamethylene, and an especially convenient example is pentamethylene. It is especially desirable for R" to be meta or para phenylene.

In a number of especially convenient embodiments, the peracid is a diperacid according to formula 1, R is hydrogen, a and d are each 1, R' and R"' are each pentamethylene, and R" is meta or para phenylene and in other embodiments the peracid is the corresponding monoperacid.

Other peroxyacids which can be contemplated for production in solid form according to the present invention comprise the polar amide-containing monoperoxyacids of USP 4686063 (Burns) to the extent that such monoperoxyacids are sufficiently stable for them to be produced and recovered and the amido monoperoxyacids of USP 5098598 (Sankey et al).

The dilution process of the peroxyacid-containing reaction mixture is often conducted at a temperature of from 0°C to 30°C.

The term effective amount of surfactant relates to an amount of surfactant which results in a detectable improvement in the dilution process in comparison with an otherwise identical, but surfactant-free dilution process. The amount of surfactant contemplated in the present invention is normally at least 0.01%. Herein, unless otherwise expressly stated, a reference to the % for the surfactant is by weight based on the weight of diluted reaction mixture. In many embodiments of the present invention process, the amount of surfactant is at least 0.02%, and in a number of advantageous embodiments is chosen in the range of 0.03 to 0.5%. In general, the amount of surfactant is usually less than 5% and in many instances up to 1%. The extent of improvement in the process tends to increase, though non-linearly with increase in concentration of the surfactant.

The extent of improvement of the surfactant-containing process over a surfactant-free process is also influenced by the particular dilution method that is selected. As a practical consequence of a substantial fraction of the benefit being attainable in at least certain process variations with relatively low surfactant concentrations, there is a preference towards employing them rather than higher concentrations of surfactant that are feasible, taking into account the marginal benefit obtained from any increase in surfactant concentration and the increased likelihood of degradation products in the product if diluent solutions containing higher levels of surfactant are recycled.

An improvement in the dilution process arising from incorporating the surfactant can manifest itself in several ways.

It can result in a decrease in the time needed to recover by filtration or like methods a desired proportion of solid product and/or the time needed to wash that product to a desired purity level. Alternatively, or additionally, the improvement can comprise a reduction in the relative volume of wash liquor needed to attain a benchmark level, often the pH of the effluent liquor. Alternatively or additionally, smaller scale plant can be employed for the peracid recovery to a desired sulphate impurity level from a peracid production plant of given scale. One further benefit that has been observed in at least some instances is that the resultant cake obtained in the same apparatus can have a higher solids content and correspondingly lower residual liquor content than a cake produced in a surfactant-free dilution process enabling a higher fraction of the liquor to be recycled and/or reducing the extent of drying needed for a dry powder product. These represent either operational or capital savings for the process. In yet a further benefit, an aqueous suspension of peroxyacid precipitated in accordance with the present invention can exhibit a lower viscosity compared with a suspension of the same weight proportion of peracid that has been precipitated in the absence of surfactant. Such advantages have been particularly noticed in the context of peroxyacids that accord with formula 1 hereinbefore.

By virtue of the employment of the invention process, and by selection of the surfactant and its concentration, it has been found possible to control the mean particle size and particle distribution of the resultant recovered solid peracid product, and particularly in respect of peracids which accord with formula 1. Where rapid dilution of the peracid-containing reaction mixture with water produces a product that has a very low particle size, as for example can arise for peracids of formula 1, which can have a mean particle size of below 2 µ, the presence of surfactant can result in a markedly increased mean particle size of the peracid precipitate and/or also in a tighter particle size distribution. By selection of the surfactant, its amount of incorporation and the quenching process, the production of formula 1 peracids having a mean particle size of over 5µ, such as in the range of 5 to 40µ have been observed. Without wishing to be bound to any theory, it is postulated that the observed increase in particle size and/or tightness of particle distribution can be linked with other observed physical benefits observed for products obtained from the invention process, including improvement in filtration or washing rates.

The surfactant can be selected from ionic and non-ionic surfactants, including conveniently, anionic or cationic surfactants. In particular, the surfactants can be selected from amongst the ranges of surfactants that have been incorporated in or proposed for incorporation in washing compositions, such as those intended for laundry purposes. Without being exhaustive, the anionic surfactant can be selected from natural and synthetic anionic detergents and normally comprise alkaline salts, particularly alkali metal salts, often sodium salts that are water soluble or dispersible of compounds containing a hydrophobic moiety and a carboxylic acid, sulphate or sulphonic acid or a phosphate moiety. Amongst synthetic detergents are included especially alkyl aryl sulphonates, such as alkylbenzene and alkyl naphthalene sulphonates having a total carbon content or from 14 to 26 carbons and particularly 17 to 20 carbons. The alkyl substituent in alkyl aryl sulphonates often contains from 8 to 18 linear carbons and in many instances is a mixture of mainly C10 to C14, averaging about 12 linear carbons, ie dodecyl.

Other suitable synthetic detergents comprise alkyl sulphates and sulphonates, sulphated derivatives of glycerides or of ethers or of alkyl succinates, phosphate ester surfactants, sucrose esters, carboxylic acid soaps and fluorinated analogues of the foregoing anionic surfactant subclasses, in many instances containing an alkyl moiety, often unbranched, having from 8 to 22 carbons. In a number of suitable instances, an alkyl moiety present in any of the foregoing anionic surfactants.is derived from tallow or coconut fatty acids or from olefins of similar molecular weight. The alkyl sulphates can optionally contain an ethoxylate moiety, often from 1 to 6 moles per mole fatty alcohol. Yet other suitable anionic surfactants include olefin sulphates and sulphonates, often derived from olefins containing from 12 to 18 carbons. Amongst the range of natural anionic detergents, a well known suitable class comprises alkali metal and especially sodium and/or potassium salts of fatty carboxylic acids, and particularly those containing from 10 to 22 carbons, such as tallow or coconut fatty acids.

The nonionic surfactants suitable for employment in the present invention can be selected in many instances from condensates of a fatty acid, or fatty alcohol, or fatty amide or alkylphenol with a polyalkyleneoxide, especially polyethyleneoxide, ie forming esters, ethers or amides respectively. In such compounds, the fatty moiety usually contains from 8 to 24 carbons and the alkylphenol moiety usually contains from 12 to 24 carbons, of which the alkyl substituent provides from 6 to 18 and often averages 9. The polyalkylene oxide moiety on average often contains from at least 4 to 20 units and in many instances from around 8 to around 15 units on average. To reduce foaming arising from the employment of glycol terminating surfactants, they can be tipped with a low molecular ester, e.g. methoxy- or ethoxy-ethylene glycol or condensed with butylene oxide. Alternatively, the hydrophilic moiety in the surfactant can be provided, at least in part, by natural polyols derived, for example from aldoses, such as glykitols. Examples of such materials include mannitol and sorbitol. The glykitols can be pre-reacted with glycols before reaction with the fatty moiety.

A further range of nonionic surfactants comprises block condensates of polypropylene glycol with polyethylene oxide, often having a molecular weight in the region of 800 to 1200. Yet further types of nonionic surfactants which can be contemplated for use in a process of the present invention are based on fatty amine oxides, though tempered by foam control desires.

The surfactant can also be selected from cationic surfactants containing a hydrophobic alkyl or aralkyl moiety, often containing from 8 to 22 carbon atoms and a quaternary ammonium or pyridinium group. The remaining quaternary substituents are often methyl, ethyl or propyl groups. Surfactants containing a cationic group include a counter-ion. In order to improve stability to in situ decomposition of peroxygen compounds, it is preferable for the counterion to be halide-free, or at least halide diminished.

A further class of surfactants that can be contemplated in the present invention comprises zwitterionic/amphoteric surfactants. Such compounds are often based upon alkyl (C8 to C20) substituted imidazolines condensed with a carboxylate or sulphonate substituent, though it is preferable to select amphoteric surfactants with lower rather than higher foam generating capabilities.

It is desirable for at least a fraction of the surfactant employed in the invention process to be selected from the classes of surfactants which either suppress foaming or are comparatively poor foam producers, such as most non-ionic surfactants and the salts of fatty carboxylic acids, particularly when implementation is contemplated on a bulk scale involving agitation and/or high pumping rates. Alternatively or additionally, it is possible to employ a foam suppressant such as those based on a silicone.

Without being bound by any theory as to mechanism by which the improvements to the product recovery process arise, it is believed that the habit or structure of the solid product is modified by the presence of the surfactant, which leads to a modification of the external shape, size and distribution of the solids that precipitate out of and grow within the reaction mixture. This in turn manifests itself in a modification of the filtration and washing properties of the solid product.

In principle, the incorporation of surfactant and diluent with peracid containing reaction mixture can be carried out in a variety of different ways. For example, the surfactant mixture can be introduced into the reaction mixture, such as by incorporating the surfactant into diluent water or by separately introducing the surfactant and diluent water solution into the reaction mixture or by introducing reaction mixture into a surfactant-containing solution. It has been found that the manner of surfactant incorporation and dilution can affect the properties of the resultant isolated solid product, as regards, for example its filterability and/or rate of washing.

At relatively high concentrations of surfactant in the diluted reaction mixture, for example of greater than 1%, it has been found that there is little difference in isolated solid products arising from the manner of introduction of the surfactant, at least for those surfactants which are capable of demonstrating a detectable improvement in solid product properties at much lower concentrations. However, the importance of selecting more or most suitable manner of introduction of surfactant increases as the surfactant concentration in the diluted reaction mixture decreases. Especially at very low concentrations, of for example at or below 0.1%, it is advantageous to add the surfactant mix to the reaction mixture. It will be understood, however, that irrespective of the variations in which the surfactant and reaction mixture are brought into contact, the surfactant is present at the time of precipitation in the dilution process and accordingly is also present during subsequent growth of the peracid crystals.

The presence of surfactant modifies the morphology of the particulate peroxyacid precipitate. It will also be understood that the larger particles of mean size over 5µ obtained following the quench process of the present invention may be recovered in the form of rods or plates, depending amongst other matters on the choice of surfactant employed. An alkyl polyglycol ether carboxylic acid encourages plate production whereas a quaternary ammonium methyl sulphate encourages rod formation. The two forms tend to have differing viscosities and stabilities of suspension at the same mean particle size, the plates tending to produce less viscous solutions and faster settling. The form of product adopted from using a surfactant quench can be observed readily using a scanning electron microscope. Such products of larger mean particle size may still exhibit the crystal structure of the smaller particles that would be produced without the benefit of surfactant being present, as shown by ¹³C NMR. However, as the surfactant concentration increases, it has further been found, in at least some instances, to modify the crystal structure itself, such that oblong particles ( blocks or rods) have been formed having a propensity to form clusters. It is postulated that, as a result of such formations, the filtration and/or wash rates can be accelerated over the particles formed from the same peracids but in single stage dilution processes in the absence of surfactant and that the viscosity of slurries containing the peroxyacid particles is altered too. It will be recognised that a fraction of the peroxyacid product, even when precipitated and grown in the presence of surfactant, may adopt the crystal structure obtained in a surfactant-free manufacturing process, ie a "standard" structure.

For terephthaloyl diamidoperoxycaproic acid of formula HO₃C-(CH₂)₅-NH-CO-C₆H₄-CO-NH-(CH₂)₅-CO₃H which is sometimes called by its abbreviation, TOPCAP and which is in accordance with formula 1 described herein before, the new crystal structure obtained in the presence of surfactant is seen to differ from the "standard" product already obtained, for example in Example 8 of publication WO 90/14336 by virtue of different spectra obtained by NMR and infra-red techniques. The new crystalline product obtained in the presence of surfactant is characterised by significant and sharp chemical shifts in ¹³C solid state NMR at 171.2 and 165.12 ppm, in comparison with standard product which has sharp chemical shifts at 175.3[9] and 167.8 ppm. The infra-red spectrum for the new crystalline form of TOPCAP shows additional, strong peaks at 3400 and 3160 cm⁻¹ which characterise the new form of product in comparison with standard product and also show a change in intensity for peaks at 1765 cm⁻¹ which is significantly stronger and at 1735 cm⁻¹ which is significantly weaker that in the spectrum for the standard product. Other, though quite weak infra red peaks can be detected at 1100, 1120 and 1140 cm⁻¹ compared with the standard product.

For formula 1 peroxyacids, the new crystalline product formed in the presence of surfactant during the recovery of peracid in solid form from the reaction mixture is characterised by a downward displacement of from 4 to 6.5 ppm in the ¹³C solid state NMR chemical shift attributable to the C in CO₃H in the region of 170 to 180 ppm in comparison with the standard crystalline peroxyacid product obtained in the absence of surfactant, the displacement in such diperacids typically being 4 to 4.5 ppm and in such monoperacids typically being 6 to 6.5ppm. For formula 1 monoperacids, which contain a carboxylic acid group as well as a percarboxylic acid group, the new crystalline product obtained in the presence of surfactant is further characterised by a downward displacement of from 5 to 6 ppm in the ¹³C solid state NMR chemical shift attributable to the C in CO₂H in the region of 170 to 180 ppm in comparison with the standard crystalline peroxyacid product obtained in the absence of surfactant

It will be recognised that the physical stability of a suspension of peracid solids in a liquid medium varies inversely with the particle size of those solids. By employment of a process according to the present invention with selection of surfactant and concentration in accordance with the disclosure herein, formula 1 peracids, di or mono, can be obtained having a particle size of at least 5µ, and in some instances in the range of 10 to 40µ. A preferred product taking into account the benefits of improved filtration rate whilst retaining suspensibility in an aqueous medium comprises formula 1 peracids having a mean particle size of from 10 to 20µ, and particularly 10 to 15µ.

In many instances, it is convenient to measure the A value (as defined hereinbefore) of the mixture as an indicator of dilution, and by corollary of when to stop diluting. The reaction mixture is usually diluted to an A value of between 0.25 and 0.6 and in many instances from 0.3 to 0.55, the precise A value depending at least in part on the value at which the peracid has been substantially recovered from solution into solid form, in part on the viscosity requirements of the user and in part upon the extent to which the peracid manufacturer wishes to recycle the spent reaction mixture. Advantageously, the use of a surfactant during the dilution process according to the instant invention provides more freedom to the process user to produce a diluted composition having an A value at the higher end of the range, ie select an A value in the range of from 0.4 to 0.5, but have an acceptable viscosity.

The dilution process is often conducted in a batch-wise manner, but can also be conducted continuously. The time taken to complete dilution is usually a function of the type of dilution process selected. In a continuous process or related batch process in which a stream of the reaction mixture is mixed with a stream of diluent solution containing the surfactant, as for example in a flow through reactor or in a reaction vessel, the dilution is effected virtually instantaneous, but in practice there is an average residence time for the diluted mixture in the chosen dilution apparatus, that can range from seconds, e.g. 5 or more through to 20 minutes. In other embodiments, particularly in batch treatments, the dilution time is governed by the rate at which conventional pumping apparatus can deliver the fluids to a dilution vessel and the number of process stages. On a small scale, the minimum is often measured in seconds, but on a scale of tonnage manufacture or larger, the minimum is often at least 1 minute and usually not more than 40 minutes.

The dilution of the reaction mixture with an aqueous medium can be conducted in a number of different ways. In one, and possibly the simplest way, the aqueous medium is introduced into the reaction mixture, normally with mixing or agitation means to promote formation of an homogeneous liquid phase at a substantially similar rate throughout the period of dilution until a desired extent of dilution has occurred. In an alternative method, the reaction mixture is introduced into a pre-calculated quantity of diluent solution. In such single stage dilution processes, the rate of dilution depends upon the rate at which fluid can be pumped into and mixed with the recipient fluid.

In some other methods of diluting, for example of diluent into reaction mixture, the rate of dilution is varied during the course of the dilution process, for example by employing a rapid dilution rate until the point is reached at which precipitation begins, followed by a slow rate during crystal growth (digestion) and then at a rate that can be either slow or preferably fast or intermediate thereafter. In this variable rate method, in the first stage of dilution, the rate of dilution, as measured by the change in A value of the mixture is generally at least 0.025 A value units per minute and in many instances is from 0.05 to 0.2 A value units per minute. The dilution period in the first stage often lasts from 5 seconds to 2 minutes, but the minimum duration can increase when large volumes of diluent are introduced in accordance with chemical engineering practice.

The subsequent dilution, in this variation of the dilution process, can be carried out in at least one further stage, and in some particularly desirable embodiments in two stages, the rate of dilution being slower in the second stage than in the first stage. The second stage slow dilution applies particularly to the period from initial precipitation of some peracid to the point at which most of and especially substantially all the peracid that is capable of being precipitated has, in fact, precipitated. Desirably, the slow rate of dilution of the second stage is continued until at least 90% and especially until at least 95% of the precipitatable peracid has precipitated. One convenient way of defining the starting and finishing points for the second and slow dilution stage is with reference to the A value of the mixture and hence the slow stage lasts for the period taken to dilute from the one a value to the second. Such difference in A values is often rather small compared with the total overall extent of dilution, but the value of controlling the dilution during that period is high. The second stage A values difference is often selected in the range of from 0.03 to 0.06 A value units. It will be understood that it is possible that the second stage dilution rate can, if desired, be continued until the composition has reached its intended extent of dilution or alternatively a different dilution rate can be employed after the composition has attained an A value at which at least 90%, preferably at least 95% of the precipitatable peracid has precipitated.

During the second stage of dilution, the rate of dilution is slower than in the first stage and often, though not exclusively at least 5 times slower. In a number of desirable embodiments the rate of lowering of the A value is not greater than 0.02 A value units per minute and in many instances the rate is selected in the range of from 0.005 to 0.015 A value units/minute.. In practice, the diluent in the second dilution stage or stages is often water, dilute hydrogen peroxide or a dilute solution having an A value of up to 0.3. When slow dilution is occurring, it is especially desirable to prevent the reaction mixture exceeding around 15°C and particularly not exceeding 10°C.

In a preferred method of operation of the dilution process, the slow second stage rate of dilution lasts solely during the period of the main precipitation of the peracid and subsequent dilution is made at any convenient rate. This rate can, if desired, be similar to the rate of the first dilution stage, or can be an intermediate rate between the two preceding stages. This variation enables the advantage of the two stage process to be retained whilst accelerating the later part of the dilution, thereby improving plant utilisation and improving operating costs. Without being bound by any theory, it is believed that the rate of change of peroxyacid solubility with change in A value is very noticeable in the narrow region below the A value at which precipitation is noticeable on dilution, so that most of the peracid is precipitated within a narrow change in A value, but tends to be much smaller at changes in lower A values of for example below about 0.5 A value. On the basis of the experimentation in the programme leading to the instant invention, it was deduced that the tendency for impurity formation is especially sensitive for peracid that is present in solution ie at a high A value and decreasingly sensitive for peracid that has precipitated out of solution and is in the presence of lower A value solution.

The rate of dilution in the second stage for the passage from the A value for onset of peracid precipitation to its substantial completion is often controlled so that the period of the second stage is selected in the range of from 5 to 20 minutes.

Preferably, the rate of dilution in the third stage is at least half up to the rate of dilution in the first stage. The rate of dilution in the third stage, where it is higher than in the second stage, can be attained by a gradual increase of the dilution rate or may be attained in a step change. In many instances, the duration of the third stage is selected in the range of from 1 to 20 minutes.

In a number of operating embodiments, and especially when employing preferred diperoxyacids of formula 1, the A value of the first stage diluted mixture is selected in the range of from 0.55 to 0.65.

It will be recognised that an alternative way of regarding the instant invention comprises the concept of a fast overall dilution in which the rate of dilution is slowed significantly during the period of precipitation of the peracid.

In a further set of variations, the dilution process is carried out by first diluting reaction mixture into a fraction of the total amount of diluent solution, ie increasing A value in the diluent solution up to an intermediate A value and subsequently diluting the remainder of the diluent solution into the partly diluted reaction mixture, thereby lowering the A value. A convenient point at which to pass from one stage to the next comprises the A value for the mixture at which precipitation would commence, which for a compound like terephthaloyl diamidoperoxycaproic acid is an A value of about 0.6. Such a combination of process steps enables the initial dilution to be conducted by introducing the more acidic solution into the less acidic solution, but also to control the rate of subsequent reduction of A value in the reaction mixture during the crystal growth phase of the precipitation, ie in the region of A value fall from 0.6 to 0.55. Whilst it will be understood that the rate of each step in this latter combination is at the discretion of the user, it is particularly desirable to conduct the first step very rapidly.

The dilution process can be carried out in conjunction with external cooling, if desired. This can be effected by traditional means, such as for example a cooling jacket around the vessel in which dilution is occurring or the immersion of cooling coils within the mixture. Alternatively or additionally, the dilution medium and/or the reaction mixture can be cooled before the dilution process commences. In many instances, the dilution process is conducted at a mixture temperature that is within or brought to a temperature of from 0 to 30°C, and in a number of embodiments at from 5°C to ambient (around 22°C).

In a further variation, the dilution process can be conducted, if desired, using at least one diluent containing added hydrogen peroxide, either in the presence or absence of sulphuric acid. The peroxide can be added in either or all dilution stages. By so doing, it is possible to prevent to some extent re-equilibration of the peroxyacid species that remain in solution and consequently improve useful peroxyacid recovery. The concentration of hydrogen peroxide in the diluent or diluents is preferably no higher than the concentration that would be employed in the reaction mixture on recycle of the recovered diluted reaction medium. In a number of embodiments the peroxide is added at a concentration of from 0.5 to 5% w/w. It is advantageous to recycle such diluted reaction medium and especially if hydrogen peroxide has been added in the dilution stage(s) since by so doing there is a reduced need for wasteful and costly disposal of reactants/reaction medium. In the presence of added hydrogen peroxide it is preferable to avoid high operating temperatures if the reaction medium is concentrated, ie by water removal, before it is recycled.

Dilution of a highly acid reaction mixture with water or an aqueous diluent to precipitate particulate peracid results in some impairment of the peracid purity and the manner of dilution affects the particle characteristics of the product. However, by employing controlled dilution in the process of the present invention and especially because it is in conjunction with precipitation in the presence of a surfactant, it is possible to control the extent of impairment of the of the purity as measured by avox content and proportion of di to monoperoxyacid (for diperoxyacid products), whilst producing the product in a particulate form that can be filtered and washed reasonably quickly. Further, the controlled single stage or multi stage route can assist in the production of a peroxyacid product with a narrower particle size distribution, which can be of benefit for controlling the rate of dispersion of the product.

It will be recognised that the spent reaction mixture separated from the solid peracid product can be recycled, at least in part by appropriate make up with fresh sulphuric acid to enable it to be employed for the further production of peracid, and that recycle is facilitated by the spent mixture having a relatively high A value, thereby reducing the need for water removal before recycling. Although any residual surfactant may be taken into account in determining the amount to employ in a subsequent recycle, it is often convenient to discount recycled surfactant, especially if very low concentrations of surfactant are contemplated.

Having described the invention in general terms, specific embodiments thereof will be described in greater detail by way of example only.

### Comparisons A and B

In these comparisons not according to the present invention, a reaction mixture containing HO₃C-(CH₂)₅-NH-CO-C₆H₄-CO-NH-(CH₂)₅-CO₃H (terephthaloyl diamidoperoxycaproic acid - TOPCAP) was obtained by introducing a solution of the corresponding dicarboxylic acid (20g) in 98% sulphuric acid (40g) into a solution of hydrogen peroxide (85% w/w, 20.4g) in sulphuric acid (36g) and ice (23.6g) over a period of 30 minutes, the reaction mixture being kept to ambient (23-25°C) by a water jacket/bath to produce a reaction mixture having an A value of 0.725.

The resultant reaction mixture containing the peroxyacid was then split into two halves, each of which was subjected to a different dilution and washing process. In Comparison A, the product solution (110g) in a stirred flask was quenched by the addition within a few seconds of ice, 75g, to produce a dilute mixture having an A value of 0.294. A solid precipitate was observed which was filtered off using a filter funnel of approximate diameter 9 cm fitted with Whatman 541 filter paper under suction from a standard water pump. Filtration took 4.5 minutes. A sample of 10 grams of the solids was washed with 800 mls of laboratory demineralised water in the same equipment. The washing took 23.8 minutes and achieved pH 1.9. The solids were then washed again using a further 800 mls of water, which took a further 45 minutes and achieved pH 4. The resultant double washed product was dried and on analysis had a purity of 89% (its measured avox content) was 6.68%, being 89% of the theoretical avox content of 7.55% for 100% pure TOPCAP.

In Comparison B, the same weight of product solution (weight) was quenched by the slow introduction into the product solution in a stirred flask of the same weight of iced water as employed in comparison A, employing a peristaltic pump and an introduction period of 60 minutes. After 20 minutes addition of diluent, precipitation of solids was observed. The solids were recovered using the same equipment as in Comparison A, and thereafter a 10g sample was twice washed with 800 mls water in the same equipment as in comparison A. The initial filtration tool< 2.5 minutes and the subsequent washings took respectively 14.2 minutes and 32 minutes. All of these times are significantly quicker than in Comparison A, demonstrating that the dilution route of comparison B produced a product that was more readily filtered and washed. Analysis of the dried product indicated that the purity was only 81%, (Avox of 6.11%), an excessive and undesirable impairment in purity.

In the Examples below, a solution of TOPCAP in an acidic reaction mixture was made by one of preparative methods P1, P2 or P3 outlined below.

### Preparative Method P1

In this method, a Caro's acid solution was prepared from H₂SO₄ solution (98% w/w, 15.4g) and ice, 1.56g at 20°C, and H₂O₂ solution (35% w/w, 21.9g) was introduced slowly over a period of about 20 minutes, with cooling to keep the temperature of the reaction mixture at below 25°C. TOCAP (10g) was dissolved in concentrated sulphuric acid solution (98% w/w, 30g) and the resultant solution (40g) was introduced dropwise with constant stirring into the Caro's acid solution over a period of between 20 and 30 minutes, whilst maintaining the reaction temperature at approximately 25°C. The reaction mixture was subsequently cooled over a period of about 5 minutes to approximately 5°C.

### Preparative Method P2

In this method, a Caro's acid solution was prepared from H₂SO₄ solution (98% w/w, 15.4g) and ice, 1.6g at 20°C, and H₂O₂ solution (35% w/w, 21.9g) was introduced slowly over a period of about 20 minutes, with cooling to keep the temperature of the reaction mixture at below 25°C. "Particulate TOCAP that retained on a standard 350µ sieve (10g) was introduced progressively with stirring into the Caro's acid solution over a period of about 5 minutes, followed by concentrated sulphuric acid solution (98% w/w, 30g) slowly over a period of 20 to 30 minutes, maintaining the temperature at about 25°C during both additions. The reaction mixture was subsequently cooled to over a period of about 5 minutes to approximately 5°C.

### Preparative Method P3

In this method, a Caro's acid solution was prepared from H₂SO₄ solution (98% w/w, 10.85g) and ice, 10.85g at 20°C, and H₂O₂ solution (70% w/w, 9.91g) was introduced slowly over a period of about 10 minutes, with cooling to keep the temperature of the reaction mixture at below 25°C. "Particulate TOPCAP that passed a standard 350µ sieve (10g) was introduced progressively with stirring into the Caro's acid solution over a period of about 5 minutes, followed by concentrated sulphuric acid solution (98% w/w, 28.4g) slowly over a period of about 25 minutes, maintaining the temperature at about 25°C during both additions. The reaction mixture was subsequently cooled to over a period of about 5 minutes to approximately 5°C.

### Preparative method P4

In this method, a Caro's acid solution was prepared from H₂SO₄ solution (98% w/w, 8.6 g) and ice, 12.2g at 20°C, and H₂O₂ solution (85% w/w, 9.2g) was introduced slowly over a period of about 20 minutes, with cooling to keep the temperature of the reaction mixture at below 25°C. TOCAP (10.0g) was dissolved in concentrated sulphuric acid solution (98% w/w, 30.0g) and the resultant solution (40.0g) was introduced dropwise with constant stirring into the Caro's acid solution over a period of between 20 and 30 minutes, whilst maintaining the reaction temperature at approximately 25°C. The reaction mixture was subsequently cooled over a period of about 5 minutes to approximately 5°C.

### Dilution of reaction mixture and precipitation of solid product

The reactions mixtures prepared by P1, P2, P3 or P4 were subsequently diluted by one of the following methods QA, QB, QC or QD.

### Dilution Method QA

In this method, a well mixed solution of a specified surfactant at a calculated concentration in water was prepared in an amount precalculated as being sufficient to dilute the reaction mixture to the desired final A value and specified surfactant concentration and was introduced in a stream into the reaction mixture with stirring during a period that lasted from 10 seconds to 2 minutes.

### Dilution Method QB

In this method, a well mixed solution of a specified surfactant at a calculated concentration in water was prepared in an amount precalculated as being sufficient to dilute the reaction mixture to the desired final A value and specified surfactant concentration. The solution was then introduced into the reaction mixture with stirring in three stages. In the first stage, which lasted a matter a few seconds, the precalculated amount of diluent was introduced to lower the A value to around 0.6 at which precipitation just was starting. In stage 2, which commenced about 1 minute later, a second portion of diluent was introduced more slowly over a period of about 2 minutes to lower the A value to about 0.55, and thereafter in the third stage remaining diluent was introduced at rather more than double the rate of the second stage and controlled to prevent the temperature of the mixture exceeding 30°C until the final A value for the mixture was attained.

### Dilution Method QC

In this method, a well mixed solution of a specified surfactant at a calculated concentration in water was prepared in an amount sufficient to dilute the reaction mixture to the desired final A value and specified surfactant concentration and the reaction mixture was introduced into the surfactant solution over a period of about 1 minute.

### Dilution Method QD

In this method, a well mixed solution of a specified surfactant at a calculated concentration in water was prepared in an amount sufficient to dilute the reaction mixture to the desired final A value and specified surfactant concentration was cooled to below 5°C and divided into two fractions. The first fraction comprised sufficient solution to dilute the reaction mixture to the A value in the region of 0.6 at which peracid precipitation commenced and the second fraction comprised the remainder. The reaction mixture was introduced with stirring over a period of about 1 minute into the first fraction of surfactant solution and cooling. The second fraction of surfactant solution was then added in a stream to the partly diluted reaction mixture over a period of 2-3 minutes.

### Dilution Method QE

In this method, a reaction mixture and a well mixed solution of a specified surfactant at a calculated concentration in water were introduced concurrently into a water-cooled jacketed vessel, initially empty, equipped with a stirrer at substantially constant rates over a period of about 35 minutes. The relative amounts of reaction mixture and surfactant solution was calculated to produce a diluted mixture having a predetermined A value and the mixture in the vessel kept at approximately ambient temperature..

The surfactant concentration in the diluent solution was calculated taking into account relative weights of the reaction mixture (starting A value of usually 072) and the diluent needed to produce the specified A value of the diluted mixture, usually 0.4 or 0.5. The calculated surfactant concentration in the diluent was about 2.5 times that in a diluted mixture at A value about 0.4 and nearly 4 times that in a diluted mixture of A value about 0.5.

After dilution in methods QA to QE was complete, the solids were recovered from the reaction mixture by filtration using a filter funnel of fitted with Whatman 541 filter paper under suction from a standard water pump. In each series of trials, a filter funnel of the same diameter was employed, except where stated, to enable a fair comparison to be made between runs. The retained solids were then washed to remove impurities by passing water through the solids retained on the filter funnel until the pH of the filtrate was pH3 by comparison with pH paper at intervals. Particularly when rapid washing was completed within a few minutes, some overshoot was observed sometimes, the effluent pH reaching a pH of from 3 to 4 before its measurement confirmed that a pH of 3 had been attained. The purity of the washed product was also measured.

The surfactants employed in the Examples, are indicated as follows:-. Nonionic
S1 - nonylphenol ethoxylate available under the trade Marl< "ETHYLAN" TU
S2 - Lauric diethanolamide available under the Trademark "CRILLON" LDE
S3 - Coconut fatty acid monoethanolamide available under the Trademark "EUMULGIN" C4
   Anionic
S4 - Linear alkyl benzenesulphonate (av Dodecyl) available under the Trademark "CAFLON" NAS30
S5 - Potassium perfluoroalkylsulphonate available under the Trademark "FLUORAD" FC98
   Cationic
S6 - Cocobenzyldimethylammonium chloride available under the Trademark ARQUAD" BSC
   Additional nonionic
S7 - Linear alkoxylate available under the Trade Marl< "ETHYLAN CD964"
S8 - Alkylphenolpolyglycol ether available under the Trade Mark "SANDOXYLATE PN-7".

### Comparison C and Examples 1 to 3

In these Examples and comparison, the reaction mixture was diluted to a final A value of 0.4, employing preparative method P4 and dilution method QB, using a 9 cm diameter filter. In the Comparison, no surfactant was employed, and the Examples, the surfactants were respectively S6, S1 and S4 to provide a final concentration in the diluted reaction mixture of 0.40% w/w.

The results are summarised in Table 1 below

**Table 1**

| Ex No | Surfactant | Filter Time mins | Wash Vol. mls | Purity % w/w |
|---|---|---|---|---|
| Comp C | none | 35 | 580 | 87.9 |
| Ex 1 | S6 | 5 | 230 | 86.9 |
| Ex 2 | S1 | 5 | 260 | 88.2 |
| Ex 3 | S4 | 5 | 250 | 86.2 |

From Table 1, it can be seen that the effect of employing each of the surfactants was to reduce substantially the filter time, and the volume of wash water needed to wash to the desired pH in the effluent, without significantly impairing the purity of the isolated solid peracid product.

The products obtained were analysed by solid state ¹³C NMR and by measurement and comparison of the peaks attributable to the C in CO₃H in the region of 170 to 180 ppm in the standard and and new forms of peroxyacid product, the weight proportions of the standard and new crystalline forms in the product were determined. For Examples 1 and 2, the proportions of new crystalline form constituted respectively 82 and 66%. In Comparison C, none of the new crystalline form was detected.

In a 40x scale repetition of comparison C, the resultant product was found to have a mean particle size of 1.75µ.

### Examples 4 to 8

In these Examples, the preparation and dilution methods were respectively P1 and QA to provide a diluted reaction mixture having a final A value of 0.5. The surfactant used was S1 at the final concentrations listed in Table 2 below, together with a summary of the results.

**Table 2**

| Ex No | Surfactant weight % | Filter diam cms | Filter Time mins | Purity % w/w |
|---|---|---|---|---|
| Ex 4 | 0.40 | 11 | 5 | 90.5 |
| Ex 5 | 0.20 | 11 | 3 | 89.1 |
| Ex 6 | 0.066 | 9 | 7 | 91.8 |
| Ex 7 | 0.033 | 7 | 11 | 87.1 |
| Ex 8 | 0.023 | 7 | 13 | 90.4 |

From Table 2, it can be seen that even very low concentrations of surfactant at the time of peracid precipitation enable fast filtration rates to be attained.

Analysis of the products obtained in Examples 4 and 6 to 8 by solid state ¹³C NMR in the manner of Example 1 demonstrated that the proportion of the new crystalline form in the product increased as the concentration of surfactant increased, comprising 10%, 23%, 43% and 74% in respectively Example 8, 7, 6 and 4. Particle size analysis for results given herein was conducted using a "Malvern Mastersizer" laser particle size analyser. The mean particle size of the product of Example 6 was about 4.5µ and that the span (the ratio of a) the 90 percentile largest diameter less the 10 percentile diameter to b) the 50 percentile diameter) was about 2.4 indicating a relatively tight distribution. Comparison with the repeat of comparison C indicates that the invention process has resulted in a significant increase in mean particle size.

### Examples 9 and 10

In these Examples, Preparative method P1 and dilution method QA were employed to produce a reaction mixture of final A value = 0.5, using Surfactant S3 at the concentrations listed in Table 3.

**Table 3**

| Ex No | Surfactant weight % | Filter Time mins | Purity % w/w |
|---|---|---|---|
| Ex 9 | 0.20 | < 5 | 88.6 |
| Ex 10 | 0.023 | 12 | 91.2 |

From Table 3, it can be seen that this surfactant was also effective at reducing the time taken for the product to be filtered.

Analysis of the product obtained in Example 9 by solid state ¹³C NMR in the manner of Example 1 demonstrated that the proportion of the new crystalline form in the product was 67% and analysis of its particle size indicated a mean particle size of 30.6µ and a span on 1.8.

### Examples 11 and 12

In these Examples, Preparative method P3 and dilution method QA were employed to produce a reaction mixture of final A value = 0.55, using respectively Surfactants S1 or S2 at a concentration of 0.066% in the diluted reaction medium.

**Table 4**

| Ex No | Surfactant | Filter diam cms | Filter Time mins | Purity % w/w |
|---|---|---|---|---|
| Ex 11 | S1 | 11 | 3 | 88.6 |
| Ex 12 | S2 | 7 | < 6 | 90.7 |

From Table 4, it can be seen that a third type of nonionic surfactant at low concentration in the diluent solution also enabled fast filtration times to be achieved.

### Examples 13 to 16

In these Examples, the preparative method was P2, followed by the different methods of dilution listed. The dilution was carried out to an A value of 0.4 using a solution of surfactant S2. The results using a 7 cms diameter filter are summarised in Table 5 below.

**Table 5**

| Ex No | Surfactant Weight % | Dilution method | Filter Time mins | Purity wt % |
|---|---|---|---|---|
| Ex 13 | 0.04 | QD | 15 | 90.7 |
| Ex 14 | 0.04 | QD | 13 | 92.1 |
| Ex 15 | 0.4 | QC | 10 | 88.7 |
| Ex 16 | 0.04 | QA | 14 | 89.8 |

From Table 6, it can be seen that a number of different dilution methods are capable of achieving fast filtration, using low or very low concentrations of surfactant.

### Example 17

In this Example, the preparative method was P1 and the quench method was QE. The concentration of surfactant S2 in the diluted solution was 0.4%. The A value of the diluted mixture was 0.5. The filter time was 10 minutes and the purity of the solids was 86.1%.

### Example 18

In this Example, 250g TOCAP was dissolved in 750g sulphuric acid (98% w/w) and the carboxylic acid solution was introduced into a preformed peroxidic solution which comprised ice/water (39g) sulphuric acid (385g, 98%) and hydrogen peroxide (847g, 35% w/w) having a temperature cooled to below 15°C. The carboxylic acid solution was introduced over a period of 30 minutes with stirring and with the temperature of the mixture was maintained with cooling below 20°C throughout. In a variation of QA, the mixture was then cooled rapidly to 5°C and quenched by the introduction with stirring over approximately 30 seconds of a preformed solution of surfactant S2 (4g) in demineralised water (694.5g). The resultant product was capable of being filtered and washed quickly and had a mean particle size of 15.6µ with a span of 1.9.

### Example 19

In this Example, a carboxylic acid solution was obtained by dissolving TOCAP (14kg) in sulphuric acid (42kg, 98% w/w) and a peroxidic solution having a temperature of 16°C by mixing sulphuric acid (21.6kg, 98%), hydrogen peroxide (30.7kg, 35% w/w) and demineralised water (2.2 kg). The carboxylic acid solution was introduced with stirring into the peroxidic solution aver a period of approximately 30 minutes during which time the temperature rose to about 27°C. The reaction mixture was cooled to about 5°C and then diluted by introduction of a solution of surfactant S1 (820g) in demineralised water (52.7kg) (dmw) which had a temperature of 10°C over a period of about 1 minute. The resultant product showed very good filtration and wash properties and had a mean particle size of 20.6µ. Analysis by ¹³C NMR showed that 94% by weight of the peracid had the new crystal form.

### Example 20

In this Example, a carboxylic acid solution was obtained by dissolving TOCAP (100g) in sulphuric acid (300g, 98% w/w) and a peroxidic solution by mixing sulphuric acid (154g, 98%), hydrogen peroxide (219g, 35% w/w) and demineralised water (15.6g). The carboxylic acid solution was introduced with stirring into the peroxidic solution aver a period of approximately 30 minutes during which time the temperature was kept at ambient laboratory temperature. The reaction mixture was cooled to about 5°C and then diluted by introduction of a solution of surfactant S7 (2.49g) in dmw (499g) over a period of about 30 seconds. The resultant product contained 76% by weight of the peracid had the new crystal form on analysis by ¹³C NMR and a mean particle size of about 4.4µ.

### Example 21

in this Example, the procedure of Example 20 was followed but at a 1/10th scale and using as quench a dispersion of surfactant S8 (0.58g) in dmw (37.7g). The resultant product had a mean particle size of 17.9µ and fast filtration was observed. Approximately 60% of the peracid comprised the new cystalline structure, measured via IR.

### Example 22

In this Example, the procedure of Example 21 was repeated but employing surfactant S1. The resultant product had a mean particle size of 12µ, contained approximately 50% of the new crystalline structure and also filtered well.

### Example 23

In this Example, a peracid was formed which contained an excess of monoperacid relative to diperacid.

A peroxidic solution was obtained by first mixing ice (14.94g) sulphuric acid (98%, 13.74g) and hydrogen peroxide (85%, 2.02g) and then particulate TOCAP (10g) was introduced with stirring. Over half an hour, further sulphuric acid (98%, 30g) was introduced at ambient temperature. The reaction was continued for a further 2 hours at ambient, and cooled to about 5°C. The reaction mixture was quenched by introduction of a solutionof surfactant S2 (0.38g) in dmw (26.6g) to produce a product which contained on the basis of avox and nmr determinations 43% w/w monoperacid, 3% TOPCAP and 54% unreacted starting material. The product was filtered easily and washed to pH3 in less than 10 minutes. Solid ¹³C NMR showed the existence in the product of new crystalline product for the monoperacid which was characterised by a chemical shift attributable to the C in CO₃H at 171.1 compared with a chemical shift of 177.5 for the same carbon in the standard crystalline peracid product that was quenched in the absence of the surfactant. Likewise the ¹³C chemical shift attributable to the C in CO₂H was at 174.3 for the new crystalline structure compared with 179.9 for the standard structure. It was determined that 13% of the monoperacid had the new crystalline structure.

From Example 23, it can be seen that the new crystalline structure is obtainable not only for diperacids in formula 1 but also for monoperacid / monocarboxylic acid products obtained from the same dicarboxylic acid substrates.

### Example 24

This Example demonstrates the relationship between a) the variation in mean particle size of TOPCAP that has been recovered in solid form by a quench process according to the present invention that employs a surfactant (as described in preceding Examples) and b) the viscosity of a suspension. 12% w/w of solid TOPCAP was suspended in water and its viscosity measure using a Brool<field viscometer. The material having a mean particle size of 7.5µ had a viscosity of 3100 cp, that of 11µ of 1100 cp and that of 20µ had 135 cp. This shows that by the use of the process of the instant invention it is possible to obtain pourable but reasonable non-settling suspensions of more than 10% w/w solid TOPCAP, provided that the process conditions are selected and controlled to produce a mean particle size product , particularly in the range of between 10 and 15µ.

### Example 25

In this Example, the process of Example 16 was repeated, but employing a mixture of surfactant S5 (0.197g) in ice (19.7g) for use as the quench. The resultant product had a purity of 88.2%, and was readily filtered and washed, washing to pH3 taking less than 7 minutes. The image on a scanning electron microscope indicated that the product was mainly rods.

## Claims

1. A process for the manufacture of particulate poorly water-soluble peroxyacid in which the peroxyacid is present in a aqueous solution of a strong acid having an A value of at least 0.6 which is brought into mixture with diluent aqueous material in an amount sufficient that the peroxyacid precipitates out of solution characterised in that contact with diluent aqueous material selected from water or dilute aqueous solution of strong acid and/or hydrogen peroxide is conducted in the presence of an effective amount of at least one surfactant.

2. A process according to claim 1 characterised in that the peroxyacid is a diperoxyacid of formula 1
HO₃C-R'-(NR)ₐ-CO-(NR)_{b}-R"-(NR)_{c}-CO-(NR)_{d}-R"'-CO₃H
or the corresponding monoperacid
HO₂C-R'-(NR)ₐ-CO-(NR)_{b}-R"-(NR)_{c}-CO-(NR)_{d}-R"'-CO₃H
in which R represents hydrogen, alkyl, aryl, or alkaryl or aralkyl containing up to 12 carbon atoms, a + b = 1, c + d = 1, R' and R"' each represents a methylene or polymethylene group of from 1 to 10 carbons that is optionally substituted by an aryl group and R" represents an arylene or a polymethylene group containing up to 10 carbon atoms, or together with an adjacent nitrogen atom an aromatic heterocyclic group.

3. A process according to claim 2 characterised in that in the peroxyacid of formula 1, a and d are each 1, b and c are each O, R' and R"' each represent a pentamethylene group and R" represents a phenylene group.

4. A process according to any preceding claim characterised in that the solution after entire dilution has an A value selected in the range of from 0.3 to 0.55 and preferably from 0.4 to 0.5.

5. A process according to any preceding claim characterised in that the amount of surfactant is selected in the range of from 0.01 to 1% by weight based on the diluted reaction medium and preferably from 0.02 to 0.4%.

6. A process according to any preceding claim characterised in that the surfactant is a nonionic, anionic or cationic surfactant.

7. A process according to claim 6 characterised in that the surfactant is selected from alkyl benzene sulphonates salts, fluorinated alkyl sulphonate salts, alcohol ethoxylates, alkylphenol ethoxylates, alkanolamides amidoethoxylates and alkyl ammonium salts.

8. A process according to any preceding claim characterised in that the dilution is carried out in a single stage.

9. A process according to claim 8 characterised in that the surfactant solution is introduced into the reaction mixture.

10. A process according to any one of claims 1 to 7 characterised in that the dilution is carried out in a plurality of stages, in the first stage of which the dilution is effected rapidly until a solid particulate precipitate is observable and thereafter in the second stage dilution is effected more slowly whilst further peracid precipitates out of solution resulting in growth of the precipitated particles.

11. A process according to claim 10 characterised in that the first stage is conducted by introducing the reaction mixture rapidly into a fraction of the surfactant solution to attain the A value at which precipitation commences and thereafter the remainder of the surfactant solution is introduced into the partly diluted reaction mixture.

12. A crystalline form of HO₃C-(CH₂)₅-NH-CO-C₆H₄-CO-NH-(CH₂)₅-CO₃H characterised by significant chemical shifts at 165.12 and 171.2 ppm in solid state ¹³C NMR spectroscopy, and by new strong peaks at 3400 and 3160 cm⁻¹ in infra red spectroscopy.

13. A crystalline form of HO₂C-(CH₂)₅-NH-CO-C₆H₄-CO-NH-(CH₂)₅-CO₃H characterised by a significant chemical shift in solid state ¹³C NMR spectroscopy at 171.1 ppm attributable to the CO₃H and at 174.3 ppm attributable to the CO₂H.

14. A crystalline form of a diperoxyacid of formula 1
HO₃C-R'-(NR)ₐ-CO-(NR)_{b}-R"-(NR)_{c}-CO-(NR)_{d}-R'"-CO₃H
or the corresponding monoperacid
in which R represents hydrogen, alkyl, aryl, or alkaryl or aralkyl containing up to 12 carbon atoms, a + b = 1, c + d = 1, R' and R"' each represents a methylene.or polymethylene group of from 1 to 10 carbons that is optionally substituted by an aryl group and R" represents an arylene or a polymethylene group containing up to 10 carbon atoms, or together with an adjacent nitrogen atom an aromatic heterocyclic group characterised by a downward displacement of from 4 to 6.5 ppm in the ¹³C solid state NMR chemical shift attributable to the C in CO₃H in the region of 170 to 180 ppm in comparison with shift observable for the C in CO₃H in the crystalline peroxyacid product obtained in the absence of surfactant.

15. A particulate diperoxyacid of formula 1
HO₃C-R'-(NR)ₐ-CO-(NR)_{b}-R"-(NR)_{c}-CO-(NR)_{d}-R'"-CO₃H
or the corresponding monoperacid
in which R represents hydrogen, alkyl, aryl, or alkaryl or aralkyl containing up to 12 carbon atoms, a + b = 1, c + d = 1, R' and R"' each represents a methylene or polymethylene group of from 1 to 10 carbons that is optionally substituted by an aryl group and R" represents an arylene or a polymethylene group containing up to 10 carbon atoms, or together with an adjacent nitrogen atom an aromatic heterocyclic group characterised in that it has a mean particle size of from 10 to 40µ.

16. A particulate peracid according to claim 15 characterised by having a mean particle size of from 10 to 15µ.

17. A particulate peracid according to claim 16 characterised in that the particles have the form of plates.

18. A particulate peracid according to any of claims 15 to 17 characterised in that the peracid has the formula
HO₃C-(CH₂)₅-NH-CO-C₆H₄-CO-NH-(CH₂)₅-CO₃H

## Patentansprüche

1. Verfahren zur Herstellung von teilchenförmiger, schlecht wasserlöslicher Peroxysäure, bei dem die Peroxysäure in einer wässerigen Lösung einer starken Säure mit einem A-Wert von mindestens 0,6 vorliegt, welche mit verdünntem wässerigen Material in einer zur Fällung der Peroxysäure aus der Lösung ausreichenden Menge vermischt wird, dadurch gekennzeichnet, daß der Kontakt mit verdünntem, wässerigem Material, ausgewählt aus Wasser oder verdünnter, wässeriger Lösung von starker Säure und/oder Wasserstoffperoxid, in Gegenwart einer wirksamen Menge mindestens eines Tensids erfolgt.

2. Verfahren nach Anspruch 1, dadurch gekennzeichnet, daß die Peroxysäure eine Diperoxysäure der Formel 1
HO₃C-R'-(NR)ₐ-CO-(NR)_{b}-R"-(NR)_{c}-CO-(NR)_{d}-R"'-CO₃H
oder der entsprechenden Monopersäure
HO₂C-R'-(NR)ₐ-CO-(NR)_{b}-R"-(NR)_{c}-CO-(NR)_{d}-R"'-CO₃H
ist, worin R Wasserstoff, Alkyl, Aryl oder Alkaryl oder Aralkyl, das bis zu 12 Kohlenstoffatome enthält, darstellt, a + b = 1, c + d = 1, R' und R"' jeweils eine Methylen- oder Polymethylengruppe mit 1 bis 10 Kohlenstoffatomen wiedergeben, die gegebenenfalls mit einer Arylgruppe substituiert ist und R" eine Arylen- oder Polymethylengruppe bedeutet, die bis zu 10 Kohlenstoffatome enthält, oder zusammen mit einem benachbarten Stickstoffatom eine aromatische heterocyclische Gruppe darstellt.

3. Verfahren nach Anspruch 2, dadurch gekennzeichnet, daß in der Peroxysäure der Formel 1 a und d jeweils 1 sind, b und c jeweils 0 sind, R' und R"' jeweils eine Pentamethylengruppe wiedergeben und R" eine Phenylengruppe wiedergibt.

4. Verfahren nach einem vorangehenden Anspruch, dadurch gekennzeichnet, daß die Lösung nach vollständiger Auflösung einen A-Wert aufweist, die im Bereich von 0,3 bis 0,55 und vorzugsweise 0,4 bis 0,5 ausgewählt wird.

5. Verfahren nach einem vorangehenden Anspruch, dadurch gekennzeichnet, daß die Menge an Tensid im Bereich von 0,01 bis 1 Gewichtsprozent und vorzugsweise von 0,02 bis 0,4%, bezogen auf das verdünnte Reaktionsmedium, ausgewählt wird.

6. Verfahren nach einem vorangehenden Anspruch, dadurch gekennzeichnet, daß das Tensid ein nichtionisches, anionisches oder kationisches Tensid ist.

7. Verfahren nach Anspruch 6, dadurch gekennzeichnet, daß das Tensid aus Alkylbenzolsulfonatsalzen, fluorierten Alkylsulfonatsalzen, Alkoholethoxylaten, Alkylphenolethoxylaten, Alkanolamiden, Amidoethoxylaten und Alkylammoniumsalzen ausgewählt ist.

8. Verfahren nach einem vorangehenden Anspruch, dadurch gekennzeichnet, daß die Verdünnung in einer einzigen Stufe ausgeführt wird.

9. Verfahren nach Anspruch 8, dadurch gekennzeichnet, daß die Tensidlösung in das Reaktionsgemisch eingeführt wird.

10. Verfahren nach einem der Ansprüche 1 bis 7, dadurch gekennzeichnet, daß die Verdünnung in einer Vielzahl von Stufen ausgeführt wird, wobei in der ersten Stufe davon die Verdünnung schnell bewirkt wird, bis ein fester, teilchenförmiger Niederschlag zu beobachten ist, und anschließend in der zweiten Stufe die Verdünnung langsamer bewirkt wird, während weitere Persäure aus der Lösung ausfällt, wodurch die ausgefallenen Teilchen wachsen.

11. Verfahren nach Anspruch 10, dadurch gekennzeichnet, daß die erste Stufe durch schnelles Einführen des Reaktionsgemisches in eine Fraktion der Tensidlösung erfolgt, um den A-Wert zu erzielen, bei dem Ausfällung beginnt, und anschließend der Rest der Tensidlösung in das teilweise verdünnte Reaktionsgemisch eingeführt wird.

12. Kristalline Form von HO₃C-(CH₂)₅-NH-CO-C₆H₄-CO-NH-(CH₂)₅-CO₃H, gekennzeichnet durch signifikante chemische Verschiebungen bei der ¹³C-NMR-Spektroskopie im festen Zustand bei 165,12 und 171,2 ppm und durch neue, starke Peaks in der Infrarotspektroskopie bei 3400 und 3160 cm⁻¹.

13. Kristalline Form von HO₂C-(CH₂)₅-NH-CO-C₆H₄-CO-NH-(CH₂)₅-CO₃H, gekennzeichnet durch eine signifikante chemische Verschiebung bei der ¹³C-NMR-Spektroskopie im festen Zustand bei 171,1 ppm für CO₃H und bei 174,3 ppm für CO₂H.

14. Kristalline Form einer Diperoxysäure der Formel 1
HO₃C-R'-(NR)ₐ-CO-(NR)_{b}-R"-(NR)_{c}-CO-(NR)_{d}-R"'-CO₃H
oder der entsprechenden Monopersäure,
worin R Wasserstoff, Alkyl, Aryl oder Alkaryl oder Aralkyl, das bis zu 12 Kohlenstoffatome enthält, darstellt, a + b = 1, c + d = 1, R' und R"' jeweils eine Methylen- oder Polymethylengruppe mit 1 bis 10 Kohlenstoffatomen wiedergeben, die gegebenenfalls mit einer Arylgruppe substituiert ist und R" eine Arylen- oder Polymethylengruppe bedeutet, die bis zu 10 Kohlenstoffatome enthält, oder zusammen mit einem benachbarten Stickstoffatom eine aromatische heterocyclische Gruppe darstellt, gekennzeichnet durch eine Verschiebung von 4 bis 6,5 ppm feldabwärts in der chemischen Verschiebung von ¹³C-NMR- im festen Zustand für C in CO₃H im Bereich von 170 bis 180 ppm, im Vergleich zu jener für C in CO₃H bei dem kristallinen, in Abwesenheit von Tensid erhaltenen Peroxysäureprodukt zu beobachtenden Verschiebung.

15. Teilchenförmige Diperoxysäure der Formel 1
HO₃C-R'-(NR)ₐ-CO-(NR)_{b}-R"-(NR)_{c}-CO-(NR)_{d}-R"'-CO₃H
oder die entsprechende Monopersäure,
worin R Wasserstoff, Alkyl, Aryl oder Alkaryl oder Aralkyl, das bis zu 12 Kohlenstoffatome enthält, darstellt, a + b = 1, c + d = 1, R' und R"' jeweils eine Methylen- oder Polymethylengruppe mit 1 bis 10 Kohlenstoffatomen wiedergeben, die gegebenenfalls mit einer Arylgruppe substituiert ist und R" eine Arylen- oder Polymethylengruppe bedeutet, die bis zu 10 Kohlenstoffatome enthält, oder zusammen mit einem benachbarten Stickstoffatom eine aromatische heterocyclische Gruppe darstellt, dadurch gekennzeichnet, daß sie eine mittlere Teilchengröße von 10 bis 40 µm aufweist.

16. Teilchenförmige Persäure nach Anspruch 15, dadurch gekennzeichnet, daß sie eine mittlere Teilchengröße von 10 bis 15 µm aufweist.

17. Teilchenförmige Persäure nach Anspruch 16, dadurch gekennzeichnet, daß die Teilchen die Form von Tafeln aufweisen.

18. Teilchenförmige Persäure nach einem der Ansprüche 15 bis 17, dadurch gekennzeichnet, daß die Persäure die Formel
HO₃C-(CH₂)₅-NH-CO-C₆H₄-CO-NH-(CH₂)₅-CO₃H
aufweist.

## Revendications

1. Un procédé pour la fabrication de peroxyacide granulaire faiblement soluble dans l'eau, dans lequel le peroxyacide est présent dans une solution aqueuse d'un acide fort, ayant une valeur A d'au moins 0.6, qui est mélangée avec un diluant aqueux en proportion suffisante pour que le peroxyacide précipite de la solution, caractérisé en ce que le contact avec le diluant aqueux, soit de l'eau soit une solution aqueuse diluée d'acide fort et/ou de peroxyde d'hydrogène, est réalisé en présence d'une dose efficace d'au moins un tensioactif.

2. Un procédé conforme à la revendication 1 caractérisé en ce que le peroxyacide est un diperoxyacide de formule 1
HO₃C-R'-(NR)ₐ-CO-(NR)_{b}-R''-(NR)_{c}-CO-(NR)_{d}-R'''-CO₃H
ou bien le monoperacide correspondant
HO₂C-R'-(NR)ₐ-CO-(NR)_{b}-R''-(NR)_{c}-CO-(NR)_{d}-R'''-CO₃H
dans laquelle R représente de l'hydrogène, alkyle, aryle, alkylaryle ou arylalkyle contenant jusqu'à 12 atomes de carbone, a + b = 1, c + d = 1, R' et R''' représentent chacun un groupe méthylène ou polyméthylène de 1 à 10 atomes de carbone qui peut être substitué en option par un groupe aryle et R'' représente un groupe arylène ou polyméthylène contenant jusqu'à 10 atomes de carbone ou un groupe aromatique hétérocyclique combiné avec un atome d'azote adjacent.

3. Un procédé conforme à la revendication 2 caractérisé en ce que dans le peroxyacide de formule 1, a et d valent chacun 1, b et c valent chacun 0, R' et R''' représentent chacun un groupe pentaméthylène et R'' représente un groupe phénylène.

4. Un procédé conforme à l'une des revendications précédentes, caractérisé en ce qu'après dilution totale la solution a une valeur A choisie dans la fourchette de 0.3 à 0.55 et de préférence de 0.4 à 0.5.

5. Un procédé conforme à l'une des revendications précédentes, caractérisé en ce que la dose de tensioactif est choisie dans la fourchette de 0.01 à 1% en poids de milieu réactionnel dilué et de préférence de 0.02 à 0.4%.

6. Un procédé conforme à l'une des revendications précédentes, caractérisé en ce que le tensioactif est un tensioactif nonionique, anionique ou cationique.

7. Un procédé conforme à la revendication 6, caractérisé en ce que le tensioactif est choisi parmi les sels d'alkylbenzène sulfonate, les sels d'alkylsulfonate fluoré, les éthoxylates d'alcool, les éthoxylates d'alkylphénol, les amidoéthoxylates d'alkanolamide et les sels d'alkylammonium.

8. Un procédé conforme à l'une des revendications précédentes, caractérisé en ce que la dilution est réalisée en une seule étape.

9. Un procédé conforme à la revendication 8 caractérisé en ce que la solution de tensioactif est introduite dans le milieu réactionnel.

10. Un procédé conforme à l'une des revendications 1 à 7, caractérisé en ce que la dilution est réalisée en plusieurs étapes, dans la première étape la dilution étant effectuée rapidement jusqu'à ce qu'un précipité de particules solides soit observé ensuite, au cours de la deuxième étape, la dilution étant effectuée plus lentement pendant que le peracide continue à précipiter de la solution en provoquant la croissance des particules précipitées.

11. Un procédé conforme à la revendication 10, caractérisé en ce que la première étape est conduite en introduisant le mélange réactionnel rapidement dans une fraction de la solution de tensioactifjusqu'à atteindre la valeur A à laquelle débute la précipitation et que le reste de la solution de tensioactif est introduit ensuite dans le milieu réactionnel partiellement dilué.

12. Une forme cristalline de HO₃C-(CH₂)₅-NH-CO-C₆H₄-CO-NH-(CH₂)₅-CO₃H caractérisée par des déplacements chimiques significatifs à 165.12 et 171.2 ppm en spectroscopie RMN ¹³C à l'état solide et par de nouveaux pics importants à 3400 et 3160 cm⁻¹ en spectroscopie infrarouge.

13. Une forme cristalline de HO₂C-(CH₂)₅-NH-CO-C₆H₄-CO-NH-(CH₂)₅-CO₃H caractérisée par un déplacement chimique significatif à 171.2 ppm en spectroscopie RMN ¹³C à l'état solide attribuable à CO₃H et à 174.3 attribuable à CO₂H.

14. Une forme cristalline d'un diperoxyacide de formule 1
HO₃C-R'-(NR)ₐ-CO-(NR)_{b}-R''-(NR)_{c}-CO-(NR)_{d}-R'''-CO₃H
ou du monoperacide correspondant
dans laquelle R représente de l'hydrogène, alkyle, aryle, alkylaryle ou arylalkyle contenant jusqu'à 12 atomes de carbone, a + b = 1, c + d = 1, R' et R"' représentent chacun un groupe méthylène ou polyméthylène de 1 à 10 atomes de carbone qui peut être substitué en option par un groupe aryle et R'' représente un groupe arylène ou polyméthylène contenant jusqu'à 10 atomes de carbone ou un groupe aromatique hétérocyclique combiné avec un atome d'azote adjacent, caractérisée par une diminution de 4 à 6.5 ppm du déplacement chimique en RMN ¹³C à l'état solide attribuable au C dans CO₃H dans la fourchette de 170 à 180 ppm en comparaison avec le déplacement qui peut être observé pour le C dans CO₃H dans le peroxyacide cristallin obtenu en absence de tensioactif.

15. Un diperoxyacide granulaire de formule 1
HO₃C-R'-(NR)ₐ-CO-(NR)_{b}-R''-(NR)_{c}-CO-(NR)_{d}-R'''-CO₃H
ou le monoperacide correspondant
dans laquelle R représente de l'hydrogène, alkyle, aryle, alkylaryle ou arylalkyle contenant jusqu'à 12 atomes de carbone, a + b = 1, c + d = 1, R' et R''' représentent chacun un groupe méthylène ou polyméthylène de 1 à 10 atomes de carbone qui peut être substitué en option par un groupe aryle et R" représente un groupe arylène ou polyméthylène contenant jusqu'à 10 atomes de carbone ou un groupe aromatique hétérocyclique combiné avec un atome d'azote adjacent, caractérisé en ce qu'il a un diamètre moyen de particules de 10 à 40 µm.

16. Un peracide granulaire conforme à la revendication 15 caractérisé par un diamètre moyen de particules de 10 à 15µm.

17. Un peracide granulaire conforme à la revendication 16 caractérisé en ce que les particules ont la forme de plaques.

18. Un peracide granulaire conforme à l'une des revendications 15 à 17 caractérisé en ce que le peracide est de formule
HO₃C-(CH₂)₅-NH-CO-C₆H₄-CO-NH-(CH₂)₅-CO₃H.
